# EUROPEAN PATENT APPLICATION

(11) **EP 4 510 083 A1**
(43) Date of publication of application: **19.02.2025**
(21) Application number: 23788480.4
(22) Date of filing: 17.03.2023
(51) Int. Cl.: G06T 17/20, G06T 19/20, G06T 7/11, G06T 7/50, G06T 7/73, G06V 10/25, A61C 9/00, A61B 5/00

(54) **METHOD, APPARATUS AND RECORDING MEDIUM STORING COMMANDS FOR PROCESSING SCANNED IMAGE OF INTRAORAL SCANNER**

(30) Priority: 14.04.2022 KR 20220046091
(71) Applicant: Medit Corp., Seoul 07207 (KR)
(72) Inventor: CHO, Young Mok, Seoul 07207 (KR)
(74) Representative: MFG Patentanwälte Meyer-Wildhagen Meggle-Freund Gerhard PartG mbB
(86) International application number: PCT/KR2023/003566
(87) International publication number: WO 2023/200128

(57) **Abstract**

The present disclosure relates to a method for processing scanned images from an intraoral scanner, an apparatus for performing the method, and a recording medium for recording instructions for performing the method. An image processing method according to some embodiments of the present disclosure, which is implemented by an electronic apparatus, may include: identifying a tooth region in a two-dimensional image of a target oral cavity; identifying, in the two-dimensional image, a first neighboring region located within a predetermined distance from a boundary of the tooth region; determining, based on a difference in depth between the tooth region and the first neighboring region, whether to include the first neighboring region in a region of interest; and generating a three-dimensional image of the target oral cavity from the two-dimensional image which includes the region of interest.

## Description

### TECHNICAL FIELD

The present disclosure relates to a method for processing a scanned image from an intraoral scanner, an apparatus for performing the method, and a recording medium in which commands for performing the method are recorded. More specifically, the present disclosure relates to a method for filtering out noise existing in a three-dimensional image generated based on a scanned image from an intraoral scanner, etc.

### BACKGROUND

A three-dimensional intraoral scanner is an optical instrument that is inserted into an oral cavity of a patient, is used for scanning of the oral cavity, and thus acquires a three-dimensional image of the oral cavity. Specifically, the three-dimensional intraoral scanner may acquire multiple two-dimensional images of the oral cavity of the patient, and the acquired multiple two-dimensional images are used for post-image processing, whereby a three-dimensional image for the oral cavity of the patient can be generated.

The generated three-dimensional image is an image based on the multiple two-dimensional images, and thus whether there is noise (e.g., a doctor's finger, a treatment instrument, a cheek soft tissue, the tongue soft tissue, etc.) represented in the two-dimensional images has a considerable effect on the quality of the three-dimensional image.

The quality of the three-dimensional image of an oral cavity of a patient has a direct effect on a series of dental treatment processes such as establishment of the patient's treatment plan, review of the patient's treatment process, and review of the patient's treatment results, and thus there is a need for a technology for generating a high-quality three-dimensional image of the oral cavity of the patient. In addition, as a technology for generating a high-quality three-dimensional image, a technology for appropriately filtering out noise represented in a two-dimensional image is required. Above all, since soft tissue, which is a type of noise, is represented by an attribute on a two-dimensional image that is similar to that of a region of interest (e.g., a tooth, the gingiva, etc.), a technology for effectively filtering out such soft tissue is required.

### SUMMARY

The present disclosure provides a method for determining a region of interest on a two-dimensional image, the region being obtained after filtering out noise, based on a difference in depth from a region represented on the two-dimensional image of an oral cavity of a patient. In addition, the present disclosure provides a method for generating a high-quality three-dimensional image of an oral cavity of a patient by using a two-dimensional image for generation of the three-dimensional image, the two-dimensional image being obtained after filtering out noise.

The technical problems to be solved in the present disclosure are not limited to the mentioned technical problems, and other unmentioned technical problems can be clearly understood by those skilled in the art from the description below.

An image processing method performed by an electronic device according to some embodiments may include: identifying a tooth region in a two-dimensional image of a target oral cavity; identifying, in the two-dimensional image, a first neighboring region located within a predetermined distance from a boundary of the tooth region; determining, based on a difference in depth between the tooth region and the first neighboring region, whether to include the first neighboring region in a region of interest; and generating a three-dimensional image of the target oral cavity from the two-dimensional image which includes the region of interest.

In some embodiments, the identifying the tooth region may include identifying the tooth region in the two-dimensional image by using a tooth segmentation model constructed according to a machine learning algorithm, and the tooth segmentation model may correspond to a model trained by modeling a correlation between a training image set of a tooth and a segmentation result image set corresponding to the training image set.

In some embodiments, the determining whether to include the first neighboring region in the region of interest may include comparing a first coordinate corresponding to the tooth region with a second coordinate corresponding to the first neighboring region to calculate the difference in depth, and each of the first coordinate and the second coordinate may correspond to a coordinate obtained using an intraoral scanner linked to the electronic device. Here, each of the first coordinate and the second coordinate may be a coordinate calculated based on a position of a first camera, a position of a second camera distinguished from the first camera, an image captured by the first camera, and an image captured by the second camera, and the first camera and the second camera may be cameras provided in the intraoral scanner. Further, each of the first coordinate and the second coordinate may be a coordinate obtained through monocular depth estimation of the two-dimensional image captured from the intraoral scanner.

In some embodiments, the determining of whether to include the first neighboring region in the region of interest may include excluding the first neighboring region from the region of interest when the difference in depth is equal to or greater than a threshold. Here, the excluding the first neighboring region from the region of interest may include even when a difference in depth between the first neighboring region and a second neighboring region located within the predetermined distance from the boundary of the first neighboring region is less than the threshold, excluding the second neighboring region from the region of interest.

In some embodiments, the determining whether to include the first neighboring region in the region of interest may include when the difference in depth is less than a threshold, including the first neighboring region in the region of interest. Here, the including the first neighboring region in the region of interest may include repeatedly expanding the region of interest until the difference in depth between the region of interest and a third neighboring region located within the predetermined distance from the boundary of the region of interest becomes equal to or greater than the threshold. Further, a distance between the boundary of the region of interest and the third neighboring region may increase as an expansion count increases. Furthermore, the repeatedly expanding of the region of interest may include when an expansion count is equal to or greater than a reference count, suspending an expansion of the region of interest even when the difference in depth between the region of interest and the third neighboring region is less than the threshold.

In some embodiments, the image processing method may further include displaying the region of interest by highlighting the region of interest on the two-dimensional image or displaying the region of interest by highlighting the region of interest on the three-dimensional image.

An electronic device according to some embodiments may include a processor, a network interface communicatively connected to an intraoral scanner, a display, a memory, and a computer program loaded onto the memory and executed by the processor, wherein the computer program comprises instructions for: identifying a tooth region in a two-dimensional image of a target oral cavity; identifying, in the two-dimensional image, a first neighboring region located within a predetermined distance from a boundary of the tooth region; determining, based on a difference in depth between the tooth region and the first neighboring region, whether to include the first neighboring region in a region of interest, and generating a three-dimensional image of the target oral cavity from the two-dimensional image which includes the region of interest.

In some embodiments, the instruction for determining whether to include the first neighboring region in the region of interest may comprise an instruction for when the difference in depth is equal to or greater than a threshold, excluding the first neighboring region from the region of interest, and when the difference in depth is less than the threshold, including the first neighboring region in the region of interest.

In some embodiments, the computer program may further comprise an instruction for displaying the region of interest by highlighting the region of interest on the two-dimensional image or displaying the region of interest by highlighting the region of interest on the three-dimensional image.

According to some embodiments, a non-transitory computer-readable recording medium in which a computer program to be executed by a processor is recorded, the computer program may comprise instructions for: identifying a tooth region in a two-dimensional image of a target oral cavity; identifying, in the two-dimensional image, a first neighboring region located within a predetermined distance from a boundary of the tooth region; determining, based on a difference in depth between the tooth region and the first neighboring region, whether to include the first neighboring region in a region of interest; and generating a three-dimensional image of the target oral cavity from the two-dimensional image which includes the region of interest.

In some embodiments, the computer program may further comprise an instruction for displaying the region of interest by highlighting the region of interest on the two-dimensional image or displaying the region of interest by highlighting the region of interest on the three-dimensional image.

### BRIEF DESCRIPTION OF DRAWINGS

FIG. 1 is an exemplary view illustrating a scanning environment according to some embodiments of the present disclosure.
FIG. 2 is an exemplary block diagram illustrating each of an electronic device and an intraoral scanner illustrated in FIG. 1.
FIG. 3 is an exemplary view illustrating the intraoral scanner described with reference to FIG. 2.
FIG. 4 is an exemplary view illustrating in detail an operation of generating a three-dimensional image of an oral cavity of a subject, which can be referred to in some embodiments of the present disclosure.
FIG. 5 is an exemplary flow chart illustrating an image processing method according to some embodiments of the present disclosure.
FIG. 6 is an exemplary view illustrating in detail an operation of identifying a tooth region, described with reference to FIG. 5.
FIG. 7 is an exemplary view illustrating an actual output image of a tooth segmentation model described with reference to FIG. 6.
FIG. 8 is an exemplary view illustrating an ideal output image of a tooth segmentation model described with reference to FIG. 7.
FIG. 9 is an exemplary view illustrating a two-dimensional image of a target oral cavity, which can be referred to in some embodiments of the present disclosure.
FIG. 10 is an exemplary view illustrating in detail an operation of identifying a first neighboring region, described with reference to FIG. 5, as an operation performed on the two-dimensional image of the target oral cavity, described with reference to FIG. 9.
FIG. 11 is an exemplary flow chart illustrating in more detail an operation of determining a region of interest, described with reference to FIG. 5.
FIGS. 12A to 12D are exemplary views illustrating in detail an operation of determining a region of interest, described with reference to FIG. 11.
FIG. 13 is an exemplary view illustrating in detail an operation of extending a region of interest and an operation of determining a neighboring region upon the extension, which can be referred to in some embodiments of the present disclosure.
FIG. 14A is an exemplary view illustrating a region of interest determined for a two-dimensional image of a target oral cavity as described with reference to FIG. 9. FIG. 14B is an exemplary view illustrating a two-dimensional image of a target oral cavity, on which a region of interest is highlighted, as described with reference to FIG. 14A.
FIG. 15 is another exemplary flow chart illustrating an image processing method according to some embodiments of the present disclosure.
FIG. 16 illustrates a screen of an electronic device, which can be referred to in some embodiments of the present disclosure, and is an exemplary view illustrating a two-dimensional image and a three-dimensional image of a target oral cavity.
FIG. 17 is another exemplary flow chart illustrating an image processing method according to some embodiments of the present disclosure.
FIGS. 18 and 19 are screens of an electronic device, which can be referred to in some embodiments of the present disclosure, and are exemplary views illustrating a three-dimensional image of a target oral cavity.

### DETAILED DESCRIPTION

Embodiments of the present disclosure are illustrated for describing the technical spirit of the present disclosure. The scope of the claims according to the present disclosure is not limited to the embodiments described below or to the detailed descriptions of these embodiments.

All technical or scientific terms used herein have meanings that are generally understood by a person having ordinary knowledge in the art to which the present disclosure pertains, unless otherwise specified. The terms used herein are selected for more clear illustration of the present disclosure, and are not intended to limit the scope of the claims in accordance with the present disclosure.

The expressions "include," "provided with," "have" and the like used herein should be understood as open-ended terms connoting the possibility of including other embodiments, unless otherwise mentioned in a phrase or sentence including the expressions.

A singular expression can include meanings of plurality, unless otherwise mentioned, and the same is applied to a singular expression stated in the claims. In addition, the terms "first," "second," etc. used herein are used to distinguish a plurality of components from one another, and are not intended to limit the order or importance of the relevant components.

The term "unit" used in these embodiments means a software component or hardware component, such as a field-programmable gate array (FPGA) and an application specific integrated circuit (ASIC). However, a "unit" is not limited to software and hardware, and it may be configured to be an addressable storage medium or may be configured to run on one or more processors. Accordingly, as examples what a "unit" may mean, a "unit" may include components, such as software components, object-oriented software components, class components, and task components, as well as processors, functions, attributes, procedures, subroutines, segments of program codes, drivers, firmware, micro-codes, circuits, data, databases, data structures, tables, arrays, and variables. In addition, functions provided in components and a "unit" may be combined into a smaller number of components and "units" or further subdivided into additional components and "units."

The expression "based on" used herein is used to describe one or more factors that influences a decision, an action of judgment or an operation described in a phrase or sentence including the relevant expression, and this expression does not exclude additional factors influencing the decision, the action of judgment or the operation.

When a certain component is described as "coupled to" or "connected to" another component, this should be understood as having meaning that the certain component may be coupled or connected directly to the other component or that the certain component may be coupled or connected to the other component via a new intervening component.

In the present disclosure, artificial intelligence (AI) refers to a technology that imitates human learning ability, reasoning ability, and perception ability and implements them with a computer, and may include the concepts of machine learning and symbolic logic. Here, the machine learning (ML) may be an algorithm technology that classifies or learns features of input data by itself. Specifically, artificial intelligence technology is a machine learning algorithm that can analyze input data, learn the result of the analysis, and make judgments or predictions based on the result of the learning. In addition, technologies that use the machine learning algorithm to imitate the cognitive and judgmental functions of the human brain can also be understood as a category of artificial intelligence. For example, technical fields of linguistic understanding, visual understanding, inference/prediction, knowledge expression, and motion control may be included in the category of artificial intelligence.

The term "machine learning" used herein may refer to a process of training a neural network model using experience of processing data. Through the machine learning, computer software may mean improving its own data processing capabilities. Here, the neural network model is constructed by modeling the correlation between data, and the correlation may be expressed by a plurality of parameters. The neural network model may derive the correlation between data by extracting and analyzing features from given data, and optimizing the parameters of the neural network model by repeating this process may be referred to as machine learning. For example, the neural network model may learn mapping (correlation) between an input and an output with respect to data given as an input/output pair. Alternatively, even when only input data are given, the neural network model may learn the relationship by deriving the regularity between given data.

The terms "artificial intelligence learning model," "machine learning model," or "neural network model" used herein may be designed to implement a human brain structure on a computer, and may include a plurality of network nodes that simulate neurons of a human neural network and have weights. Here, the plurality of network nodes may have a connection relationship between them by simulating synaptic activities of neurons that exchange signals through synapses. Specifically, in the artificial intelligence learning model, a plurality of network nodes may exchange data according to a convolution connection relationship while being located in layers of different depths. The artificial intelligence learning model may be, for example, an artificial neural network model, a convolution neural network model, or the like, but the scope of the present disclosure is not limited to the above-described examples, and it should be noted that various known neural network models are applicable to the present disclosure.

Hereinafter, embodiments of the present disclosure will be described with reference to the accompanying drawings. In the accompanying drawings, like or relevant components are indicated by like reference numerals. In addition, in the following description of embodiments, repeated descriptions of the identical or relevant components will be omitted. However, even if a description of a component is omitted, such a component is not intended to be excluded in an embodiment.

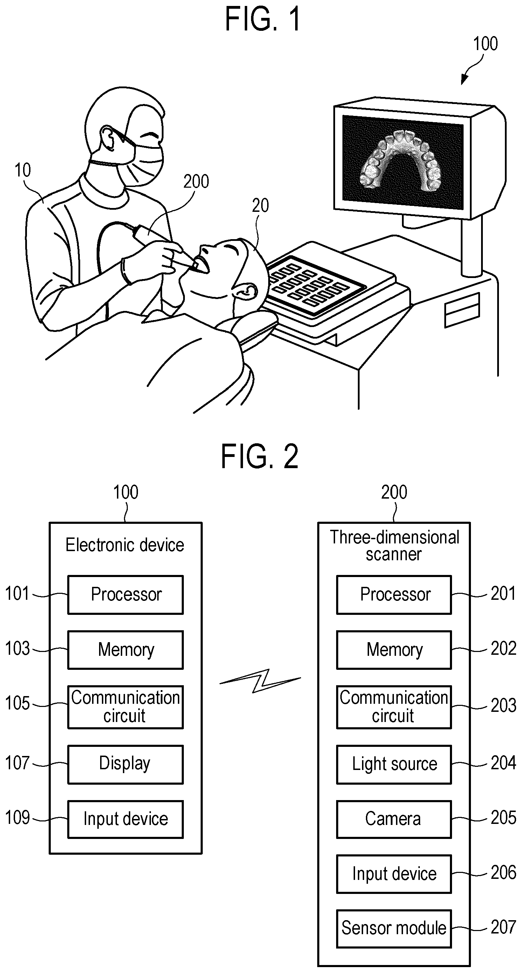

FIG. 1 is an exemplary view illustrating a scanning environment according to some embodiments of the present disclosure. Specifically, the scanning environment illustrated in FIG. 1 is an environment in which an image of an oral cavity of a patient is obtained using an intraoral scanner 200 according to some embodiments of the present disclosure, wherein the intraoral scanner 200 may be a dental instrument for obtaining an intraoral image of a subject 20 (e.g., a patient) of the intraoral scanner 200.

As illustrated in FIG. 1, a user 10 (e.g., a dentist or a dental hygienist) may obtain an image of an oral cavity of the subject 20 from the subject 20 by using the intraoral scanner 200. In another example, the user 10 may also obtain an image of an oral cavity of the subject 20 from a diagnosis model (e.g., a plaster model or an impression model) obtained by modeling after the shape of the oral cavity of the subject 20. Hereinafter, for convenience of description, a description is made based on an example of obtaining the oral cavity of the image of the subject 20, but the scope of the present disclosure is not limited to the example. In addition, a part subject to image acquisition is not limited to the oral cavity of the subject 20, and it is also possible to obtain images of other parts of the subject 20 (e.g., the ear of the subject 20).

Hereinafter, an operation of elements illustrated in FIG. 1 is described in detail.

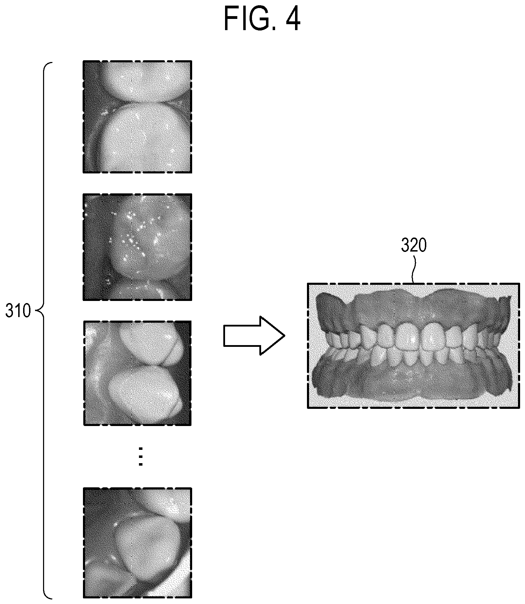

An electronic device 100 illustrated in FIG. 1 may receive a two-dimensional image of the oral cavity of the subject 20 from the intraoral scanner 200. Here, the electronic device 100 may communicate with the intraoral scanner 200 via a wired or wireless communication network. In addition, the electronic device 100 may generate a three-dimensional image of the oral cavity by performing three-dimensional modeling of an internal structure of the oral cavity, based on the received two-dimensional image of the oral cavity, and a detailed operation related thereto is described below with reference to FIG. 4. The generated three-dimensional image of the oral cavity may be displayed to the user 10 or the subject 20 through a display of the electronic device 100. In this case, the user 10 may provide a proper dental treatment service to the subject 20 with reference to the three-dimensional image displayed on the display of the electronic device 100.

In some embodiments, the electronic device 100 may receive the three-dimensional image of the oral cavity generated by the intraoral scanner 200. Here, the intraoral scanner 200 may obtain a two-dimensional image of the oral cavity by scanning the oral cavity of the subject 20, and may generate a three-dimensional image of the oral cavity based on the obtained two-dimensional image of the oral cavity. That is, it should be noted that regardless of the position where the operation of generating the three-dimensional image is processed, the operation is included in the scope of the present disclosure.

In addition, the electronic device 100 may be communicatively connected to a cloud server (not shown). In this case, the electronic device 100 may transmit the two-dimensional image of the oral cavity or the three-dimensional image of the oral cavity of the subject 20 to the cloud server, and the cloud server may store the two-dimensional image of the oral cavity of the subject 20, received from the electronic device 100, or the three-dimensional image of the oral cavity.

The above-described electronic device 100 may be implemented as a computing device, and such a computing device is described below in detail with reference to FIG. 2.

The intraoral scanner 200 illustrated in FIG. 1 may have a shape capable of being inserted into and drawn out of an oral cavity, and may be a handheld scanner which enables a user 10 to freely adjust a scanning distance and a scanning angle.

Such an intraoral scanner 200 may obtain the image of the oral cavity by being inserted into the oral cavity and scanning the oral cavity in a non-contact manner. The image of the oral cavity may include at least one tooth, the gingiva, and an artificial structure which can be inserted into the oral cavity (e.g., an orthodontic device including a bracket and a wire, an implant, a denture, an orthodontic aid inserted into the oral cavity, etc.). Specifically, the intraoral scanner 200 may emit light to the oral cavity of the subject 20 by using a light source (or a projector), and may receive light reflected from the oral cavity of the subject 20 through a camera (or at least one sensor).

In addition, the intraoral scanner 200 may obtain, as the two-dimensional image, a surface image of the oral cavity of the subject 20, based on information received through the camera. Here, the surface image of the oral cavity of the subject 20 may include at least one of a tooth, the gingiva, an artificial structure, a cheek, the tongue, or a lip.

As illustrated above, in some embodiments, the intraoral scanner 200 may obtain the two-dimensional image of the oral cavity by scanning the oral cavity, and may generate the three-dimensional image of the oral cavity based on the obtained two-dimensional image of the oral cavity.

The above-described intraoral scanner 200 may be implemented as a computing device, and an example of such a computing device is described below in detail with reference to FIG. 2.

FIG. 2 is an exemplary block diagram illustrating each of the electronic device 100 and the intraoral scanner 200 illustrated in FIG. 1. The block diagram illustrated in FIG. 2 illustrates an exemplary embodiment for achieving the objective of the present disclosure, and some elements may be added or deleted as necessary. In addition, elements of the block diagram illustrated in FIG. 2 are elements that are functionally distinguished, and it should be noted that multiple different elements may be implemented to be integrated in an actual physical environment. Hereinafter, each element illustrated in the block diagram is described in detail.

The electronic device 100 illustrated in FIG. 2 may include one or more processors 101, one or more memories 103, a communication circuit 105, a display 107, and/or an input device 109. As illustrated above, at least one of the elements included in the electronic device 100 may be omitted, or other elements may be added to the electronic device 100. In addition, additionally or alternatively, some of the elements may be implemented to be integrated, or may be implemented as a single entity or multiple entities. At least some of the elements in the electronic device 100 may be connected to each other through a bus, a general purpose input/output (GPIO), a serial peripheral interface (SPI), a mobile industry processor interface (MIPI), etc. and transmit and receive data and/or a signal.

The one or more processors 101 of the electronic device 100 may be elements capable of performing data processing or operation for control and/or communication of each element (e.g., the memory 103)) of the electronic device 100. The one or more processors 101 may be operatively connected to, for example, the elements of the electronic device 100. In addition, the one or more processors 101 may load data or a command received from other elements of the electronic device 100 in the one or more memories 103, process the data or command stored in the one or more memories 103, and store result data.

Next, the one or more memories 103 of the electronic device 100 may store various data, commands, and/or information. As a specific example, the one or more memories 103 may store instructions for operation of the processor 101 as a computer program. In addition, the one or more memories 103 may store correlation models constructed according to a machine learning algorithm. In addition, the one or more memories 103 may store data received from the intraoral scanner 200 (e.g., the two-dimensional image of the oral cavity and the three-dimensional image of the oral cavity).

Next, the communication circuit (the network interface 105) of the electronic device 100 may establish a wired or wireless communication channel with an external device (e.g., the intraoral scanner 200 and the cloud server (not shown)), and transmit and receive various data to or from the external device. In some embodiments, to communicate with the external device by wire, the communication circuit 105 may include at least one port for connection with the external device via a wired cable. In this case, the communication circuit 105 may perform communication with the external device connected to the wired cable via the at least one port. In some other embodiments, the communication circuit 105 may include a cellular communication module and may be configured to be connected to a cellular network (e.g., 3G, LTE, 5G, WiBro, or WiMAX). In some other embodiments, the communication circuit 105 may include a short-distance communication module, and may transmit or receive data to or from the external device by using short-distance communication (e.g., Wi-Fi, Bluetooth, Bluetooth low energy (BLE), or UWB). In some other embodiments, the communication circuit 105 may include a non-contact communication module for non-contact communication. Here, the non-contact communication may include, for example, at least one non-contact type proximity communication technology such as near field communication (NFC), radio frequency identification (RFID) communication, or magnetic secure transmission (MST) communication. In addition to the above-described various examples, the electronic device 100 may be implemented in various known methods for communication with the external device, and it should be noted that the above-described examples do not limit the scope of the present disclosure.

Next, the display 107 of the electronic device 100 may display various screens based on control of the processor 101. Here, based on control of the processor 101, the two-dimensional image of the oral cavity of the subject 20, received from the intraoral scanner 200, and/or the three-dimensional image of the oral cavity of the subject 20, obtained by performing three-dimensional modeling of the inner structure of the oral cavity, may be displayed through the display 107. In this case, to display the two-dimensional image and/or the three-dimensional image of the oral cavity through the display 107, for example, a web browser or a dedicated application may be installed in the electronic device 100. In some embodiments, the above-described web browser or dedicated application may be implemented to provide the user 10 with an edition function, a storage function, and a deletion function of the two-dimensional image and/or the three-dimensional image of the oral cavity through a user interface.

Next, the input device 109 of the electronic device 100 may receive a command or data to be used for an element (e.g., the processor 101) of the electronic device 100 from outside (e.g., a user) of the electronic device 100. The input device 109 may include, for example, a microphone, a mouse, a keyboard, or the like. In some embodiments, the input device 109 may be coupled to the display 107 and implemented in the form of a touch sensor panel capable of recognizing contact or proximity of various external objects. However, the scope of the present disclosure is not limited to the above-described examples, and various known input devices 109 may be included in the scope of the present disclosure for convenience of the user.

The intraoral scanner 200 illustrated in FIG. 2 may include a processor 201, a memory 202, a communication circuit 203, a light source 204, a camera 205, and/or an input device 206. As described above, at least one of the elements included in the intraoral scanner 200 may be omitted, or other elements may be added to the intraoral scanner 200. In addition, additionally or alternatively, some of the elements may be implemented to be integrated, or may be implemented as a single entity or multiple entities. At least some of the elements in the intraoral scanner 200 may be connected to each other through a bus, a general purpose input/output (GPIO), a serial peripheral interface (SPI), a mobile industry processor interface (MIPI), etc. and transmit and receive data and/or a signal.

The processor 201 of the intraoral scanner 200 may be an element capable of performing data processing or operation for control and/or communication of each element of the intraoral scanner 200, and may be operatively connected to the elements of the intraoral scanner 200. In addition, the processor 201 may load data or a command received from other elements of the intraoral scanner 200 in the memory 202, process the data or command stored in the memory 202, and store result data.

Next, the memory 202 of the intraoral scanner 200 may store instructions for the above-described operation of the processor 201.

Next, the communication circuit 203 of the intraoral scanner 200 may establish a wired or wireless communication channel with an external device (e.g., the electronic device 100), and transmit and receive various data to or from the external device. In some embodiments, to communicate with the external device by wire, the communication circuit 203 may include at least one port for connection with the external device via a wired cable. In this case, the communication circuit 203 may perform communication with the external device connected by the wired cable via the at least one port. In some other embodiments, the communication circuit 203 may include a cellular communication module and may be configured to be connected to a cellular network (e.g., 3G, LTE, 5G, WiBro, or WiMAX). In some other embodiments, the communication circuit 203 may include a short-distance communication module, and may transmit or receive data to or from the external device by using short-distance communication (e.g., Wi-Fi, Bluetooth, Bluetooth low energy (BLE), or UWB). In some other embodiments, the communication circuit 203 may include a non-contact communication module for non-contact communication. Here, the non-contact communication may include, for example, at least one non-contact type proximity communication technology such as near field communication (NFC), radio frequency identification (RFID) communication, or magnetic secure transmission (MST) communication. In addition to the above-described various examples, the intraoral scanner 200 may be implemented in various known methods for communication with the external device, and it should be noted that the above-described examples do not limit the scope of the present disclosure.

Next, the light source 204 of the intraoral scanner 200 may emit light to the oral cavity of the subject 20. For example, the light emitted from the light source 204 may be structured light having a predetermined pattern (e.g., a stripe pattern in which straight lines of different colors continuously appear). Here, the pattern of the structured light may be generated using, for example, a pattern mask or a digital micro-mirror device (DMD), but is not limited thereto.

Next, the camera 205 of the intraoral scanner 200 may obtain an image of the oral cavity of the subject 20 by receiving reflected light reflected by the oral cavity of the subject 20. Here, the camera 205 may include a left camera corresponding to a left eye field and a right camera corresponding to a right eye field to construct a three-dimensional image according to an optical triangulation method. In addition, here, the camera 205 may include at least one sensor such as a CCD sensor or a CMOS sensor.

Next, the input device 206 of the intraoral scanner 200 may receive a user input for controlling the intraoral scanner 200. For example, the input device 206 may include a button for receiving a push operation of the user 10, a touch panel for detecting a touch of the user 10, and a voice recognition device including a microphone. In this case, the user 10 may control scanning start or stop by using the input device 206.

To describe in more detail the operation of the intraoral scanner 200 controlled through the input device 206, the intraoral scanner 200 may receive a user input for starting scanning through the input device 206 of the intraoral scanner 200 or the input device 206 of the electronic device 100, and start scanning according to processing by the processor 201 of the intraoral scanner 200 or the processor 101 of the electronic device 100. Here, when the user 10 scans the oral cavity of the subject 20 through the intraoral scanner 200, the intraoral scanner 200 may generate a two-dimensional image of the oral cavity of the subject 20, and may transmit the two-dimensional image of the oral cavity of the subject 20 to the electronic device 100 in real time. In this case, the electronic device 100 may display the received two-dimensional image of the oral cavity of the subject 20 through the display 107. In addition, the electronic device 100 may generate (construct) a three-dimensional image of the oral cavity of the subject 20 based on the two-dimensional image of the oral cavity of the subject 20, and may display the three-dimensional image of the oral cavity through the display 107. In this case, the electronic device 100 may also display the three-dimensional image that is being generated in real time through the display 107.

Next, the sensor module 207 of the intraoral scanner 200 may detect an operational state of the intraoral scanner 200 or an external environmental state (e.g., the user's operation), and generate an electrical signal corresponding to the detected state. The sensor module 207 may include, for example, at least one of a gyro sensor, an acceleration sensor, a gesture sensor, a proximity sensor, or an infrared sensor. Here, the user 10 may control scanning start or stop by using the sensor module 207. In a specific example, in a case where the user 10 holds the intraoral scanner 200 with a hand and moves the same, when an angular speed measured through the sensor module 207 exceeds a configuration value, the intraoral scanner 200 may control the processor 201 to start the scanning operation.

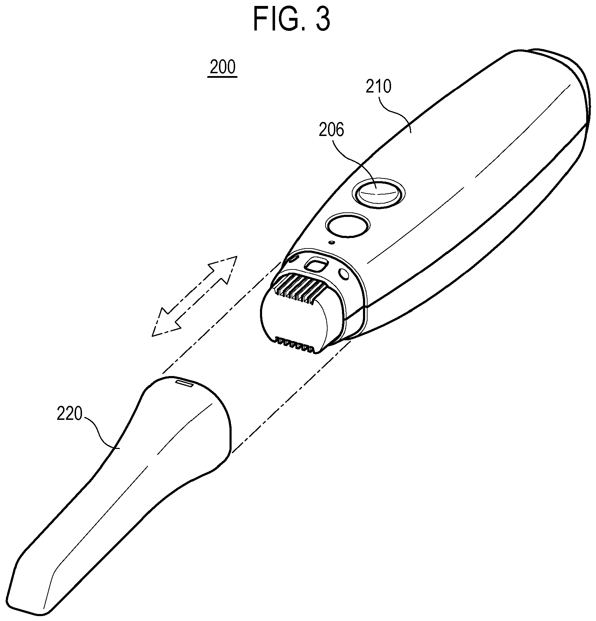

Hereinafter, referring to FIG. 3, the above-described intraoral scanner 200 is described in more detail. FIG. 3 is an exemplary view illustrating the intraoral scanner 200 described with reference to FIG. 2.

The intraoral scanner 200 illustrated in FIG. 3 may include a body 210 and a probe tip 220. Here, the body 210 of the intraoral scanner 200 may have a shape that is easy for the user 10 to grip and use, and the probe tip 220 may have a shape that facilitates insertion into and withdrawal from the oral cavity of the subject 20. In addition, the body 210 may be coupled to or detached from the probe tip 220. In addition, the above-described elements of the intraoral scanner 200 may be arranged inside the body 210. An opening may be disposed at one end of the body 210 so that light output from the light source 204 can be emitted to the subject 20. The light emitted through the opening may be reflected by the subject 20 and introduced again through the opening. Here, the reflected light introduced through the opening may be captured by a camera and an image of the subject 20 may be generated. In addition, the user 10 may start scanning by using the input device 206 (e.g., a button) of the intraoral scanner 200. For example, when the user 10 touches or presses the input device 206, light may be emitted from the light source 204 to the subject 20.

The scanning environment and the elements included therein according to some embodiments of the present disclosure are described in detail with reference FIGS. 1 to 3 above. Before describing an image processing method according to some embodiments of the present disclosure, an operation of generating a three-dimensional image of the oral cavity of the subject 20, which can be referred to in some embodiments of the present disclosure, is described below in detail with reference to FIG. 4.

FIG. 4 is an exemplary view illustrating in detail an operation of generating a three-dimensional image of the oral cavity of the subject 20, which can be referred to in some embodiments of the present disclosure. As described above, the user 10 may scan the oral cavity of the subject 20 while moving the intraoral scanner 200, and in this case, the intraoral scanner 200 may obtain multiple two-dimensional images 310 of the oral cavity of the subject 20. For example, the intraoral scanner 200 may obtain a two-dimensional image of a region including a front tooth of the subject 20 and a two-dimensional image of a region including a molar tooth of the subject 20. In this case, the intraoral scanner 200 may transmit the obtained multiple two-dimensional images 310 to the electronic device 100.

The electronic device 100 may convert each of the multiple two-dimensional images 310 of the oral cavity of the subject 20 into a set of multiple points having three-dimensional coordinate values by using the received multiple two-dimensional images 310. For example, the electronic device 100 may convert each of the multiple two-dimensional images 310 into a point cloud corresponding to a set of data points having three-dimensional coordinate values. Here, the point cloud set having three-dimensional coordinate values based on the multiple two-dimensional images 310 may be stored as raw data of the oral cavity of the subject 20. In addition, the electronic device 100 may complete the entire tooth model by aligning the point cloud, which is a set of data points having three-dimensional coordinate values.

In some embodiments, the electronic device 100 may reconfigure (reconstruct) the three-dimensional image of the oral cavity. For example, by merging the point cloud set stored as raw data by using a Poisson algorithm, the electronic device 100 may reconfigure multiple points and convert the multiple points into a closed three-dimensional surface to reconfigure a three-dimensional image 320 of the oral cavity of the subject 20. However, unlike the present example, the raw data may be processed according to various known methods, and thus it should be noted that any methods for reconfiguring the three-dimensional image of the oral cavity can be included in the scope of the present disclosure.

The operation of generating a three-dimensional image, which can be referred to in some embodiments of the present disclosure, is additionally described with reference to FIG. 4. Hereinafter, methods according to various embodiments of the present disclosure are described in detail.

Respective operations of the methods to be described below may be performed by a computing device. In other words, the respective operations of the methods may be implemented by one or more instructions executed by a processor of a computing device. All operations included in such methods may be executed by one physical computing device, but first operations of the method may be performed by a first computing device and second operations of the method may be performed by a second computing device. Hereinafter, the description is made under the assumption that the respective operations of the methods are performed by the electronic device 100 illustrated in FIG. 1. However, for convenience of description, operation structures of the respective operations included in the methods may be omitted. In addition, it should be noted that performing the respective operations of the methods by the intraoral scanner 200 illustrated in FIG. 1 is not excluded from the scope of the present disclosure.

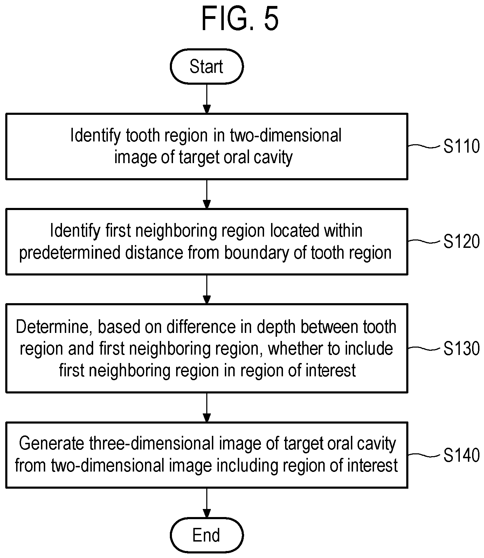

FIG. 5 is an exemplary flow chart illustrating an image processing method according to some embodiments of the present disclosure.

Referring to FIG. 5, in operation S 110, a tooth region may be identified in a two-dimensional image of a target oral cavity. Here, as described with reference to FIG. 1, the target oral cavity may mean an oral cavity of the subject 10, but the meaning of the target oral cavity is not limited to the present example, and may mean a diagnosis model obtained by modeling after the shape of the oral cavity of the subject 20. In addition, here, the tooth region corresponds to an image region included in the two-dimensional image of the target oral cavity, and may mean a region in which at least one tooth is represented.

In relation to operation S 110, the identification of the tooth region may be performed according to various methods. For example, the tooth region may be identified by directly selecting, by the user 10, the tooth region of the two-dimensional image obtained from the intraoral scanner 200. In this case, the user 10 may select the tooth region from the two-dimensional image through a user interface provided in the electronic device 100. In another example, through image processing of detecting a unique attribute of the tooth region represented in the two-dimensional image, the tooth region may be identified. Specifically, the tooth region in the two-dimensional image of the oral cavity is represented in a white-based color unlike other regions, and the tooth region may be identified through image processing of detecting a color as a unique attribute. The tooth region may be identified by other various methods, and it should be noted that any operations of identifying the tooth region from the two-dimensional image can be included in the scope of the present disclosure. Hereinafter, an operation of identifying the tooth region from the two-dimensional image by using a neural network model constructed according to a machine learning algorithm is described in more detail.

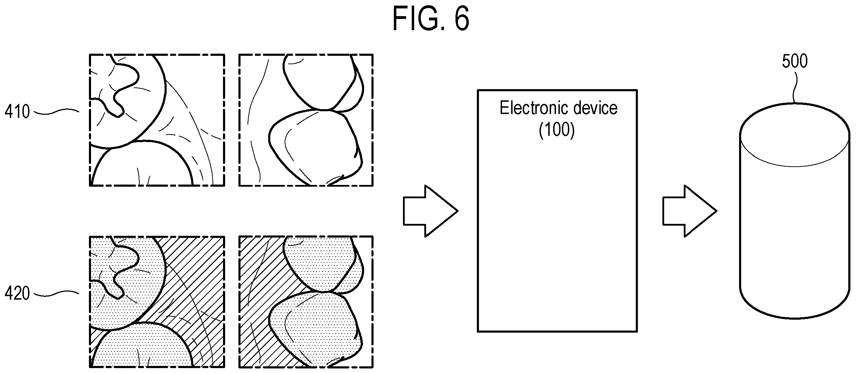

In relation to operation S 110, in some embodiments, the identifying the tooth region may include identifying a tooth region in the two-dimensional image by using a tooth segmentation model constructed according to a machine learning algorithm. Here, the tooth segmentation model may be a model trained by modeling a correlation between a learning image set for a tooth and a segmentation result image set corresponding to the training image set. A detailed description of the tooth segmentation model is described below with reference to FIG. 6.

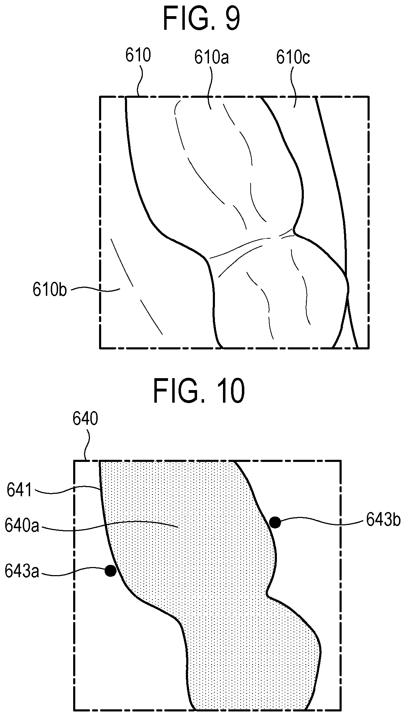

In operation S120, a first neighboring region located within a predetermined distance from a boundary of the tooth region may be identified in the two-dimensional image of the target oral cavity. Here, the predetermined distance may mean, for example, one pixel on the two-dimensional image, but the scope of the present disclosure is not limited to the present example, and the distance may change according to the implementation example thereof. That is, as described above, the tooth region is a region which can be a reference for image processing, and thus in this operation, the first neighboring region may be identified with reference to the boundary of the tooth region. A detailed description related thereto is made below with reference to FIG. 10.

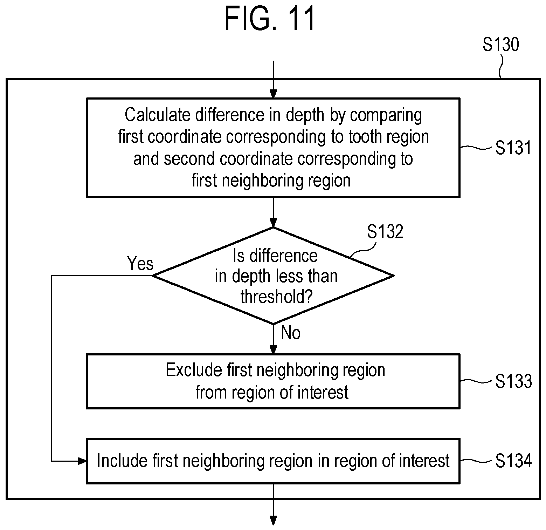

In operation S 130, whether to include the first neighboring region in a region of interest may be determined based on a difference in depth between the tooth region and the first neighboring region. Here, the depth is a value represented on an axis corresponding to a scanning direction of the intraoral scanner 200 illustrated in FIG. 1, and may mean, for example, a distance from a reference point to each region, and in this case, the reference point may be a light source 204 of the intraoral scanner 200. However, the scope of the present disclosure is not limited to the present example, and it should be noted that if a value of each region can correspond to the axis corresponding to the scanning direction, any methods can be included in the scope of the present disclosure. In addition, here, the region of interest means a region which can be a target of interest of the user 10, and for example, a tooth region and a gingiva region required for dental treatment may be a region of interest. The region of interest may be determined according to the operations to be described with reference to FIG. 11 below so that a region satisfying the intention of the user 10 may be determined, and the target of the region of interest may be adjusted by changing a configuration value (e.g., a threshold, a distance between the boundary of the region of interest and a third neighboring region, a reference count, etc.).

In operation S140, a three-dimensional image of the target oral cavity may be generated from the two-dimensional image including the region of interest. Here, the generation of the three-dimensional image may refer to the description of FIG. 4. However, the description of FIG. 4 is made according to an example of converting the entire region of the two-dimensional image into a point cloud, but unlike this, in this operation, it can be understood that the region of interest, which is a partial region included in the two-dimensional image, is converted into the point cloud.

FIG. 6 is an exemplary view illustrating in detail an operation of identifying a tooth region, described with reference to FIG. 5. Specifically, FIG. 6 illustrates an example of constructing a tooth segmentation model 500 by using a training image set 410 for a tooth and a segmentation result image set 420 corresponding to the training image set 410.

Here, the training image set 410 may mean multiple two-dimensional images of the oral cavity. In this case, the training image set 410 may mean multiple two-dimensional images randomly extracted from a two-dimensional image pool of the oral cavity of various subject groups (e.g., a male group, a female group, a group by generation, etc.). It can be understood that when a training image is extracted by limiting a two-dimensional image pool of a specific group, a training image set 410 customized for the group can be provided, and when a training image is extracted from two-dimensional image pools of various groups, a generalized training image set 410 can be provided.

In addition, the segmentation result image set 420 is an image set corresponding to the training image set 410, and may mean multiple two-dimensional images in which at least one region to be identified is masked (or segmented). In a specific example, the segmentation result image may include a tooth region mask and a gingiva region mask. However, the scope of the present disclosure is not limited thereto, the segmentation result image may further include a soft tissue region (e.g., a cheek region, a tongue region, etc.) mask, and for any region to be identified by the user 10, the segmentation result image can be provided to include a mask corresponding to the region. Here, the mask may be understood as an identifier enabling a specific region represented in the two-dimensional image to be distinguished from other regions, and may be included in the segmentation result image in the form of an image or metadata.

The segmentation result image set 420 may be generated from the training image set 410 according to various methods. For example, by overlaying masking on the training image to correspond to a user input received through the input device 109 of the electronic device 100, the segmentation result image may be generated. In addition, the segmentation result image may be generated from the training image in an automated method, and any methods of masking a specific region intended by the user 10 can be included in the scope of the present disclosure.

The tooth segmentation model 500 can be trained using the above-described training image set 410 as input data and using the segmentation result image set 420 as output data. The machine learning may be performed by the electronic device 100 illustrated in FIG. 1, but a case where the machine learning is performed by an external device and the trained tooth segmentation model 500 is loaded to the electronic device 100 is not excluded from the scope of the present disclosure.

Here, the tooth segmentation model 500 may be trained to extract various features which can be extracted from the training image, such as the texture, density, and color of the region included in the training image, the shape of the tooth, the shape of the gingiva, and the shape of the oral cavity, and derive a correlation between the segmentation result image and the training image based on the extracted features. As a machine learning algorithm which can be used for training the tooth segmentation model 500, for example, a deep neural network algorithm, a recurrent neural network algorithm, a convolutional neural network algorithm, a classification-regression analysis algorithm, reinforcement learning algorithm, or the like can be referred to, and it should be noted that all known artificial intelligence technologies for constructing the tooth segmentation model 500 by using the above-described training data having a pair of an input and an output (i.e., the training image set 410 and the segmentation result image set 420) are applicable to the present disclosure.

As described above, referring back to FIG. 6, it can be understood that the tooth segmentation model 500 can be trained by using both the training image set 410 and the segmentation result image 420 having the masked tooth region and gingiva region, as an image corresponding to the training image set 410. That is, it can be understood that the trained tooth segmentation model 500 is a model for identifying a tooth region and a gingiva region in a two-dimensional image of the oral cavity.

Referring to FIG. 6, the operation of identifying a tooth region based on an artificial intelligence model has been described above in detail. According to the above-described operation, a region intended by the user may be identified in the two-dimensional image of the oral cavity in an automated method of minimizing the involvement of the user 10. In addition, a first region (e.g., the tooth region) and a second region (e.g., the gingiva region) may be identified in the two-dimensional image of the oral cavity. That is, by filtering out other regions (e.g., a noise region) of the two-dimensional image excluding the identified regions, and generating a three-dimensional image based on the image obtained by performing the filtering, a three-dimensional image obtained by removing the noise region may be generated. For example, the three-dimensional image may be generated to include only the tooth region and the gingiva region.

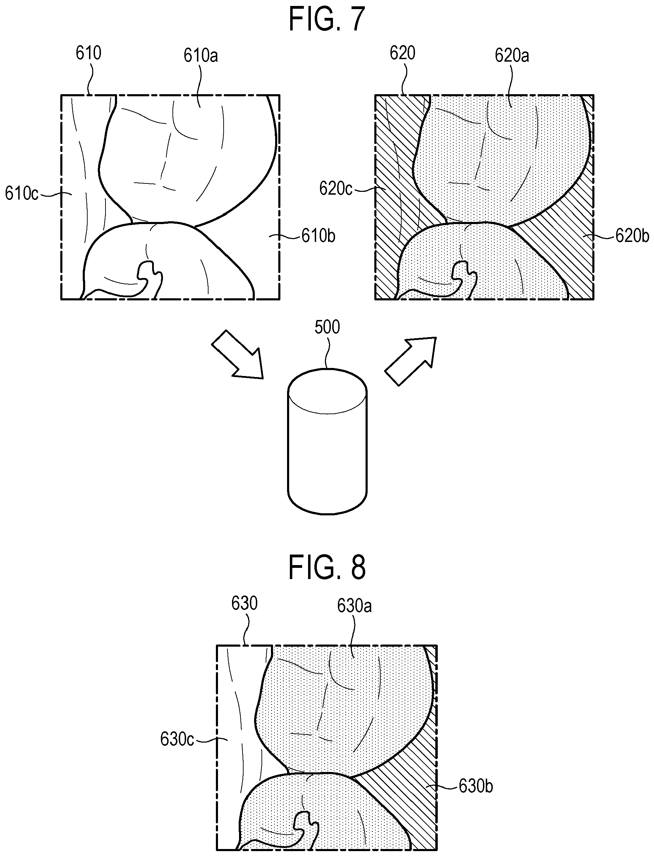

However, in an actual implementation case using the tooth segmentation model 500 trained to identify only the tooth region and the gingiva region, a soft tissue region, which can be a type of a noise region, is represented to have a similar attribute (e.g., color, texture, or the like) to that of the gingiva region on the two-dimensional image, thus the soft tissue region may be identified in the two-dimensional image differently from the intention of the user 10, and accordingly, the soft tissue region could have been included in the three-dimensional image. A detailed description related thereto is made below with reference to FIGS. 7 and 8.

FIG. 7 is an exemplary view illustrating an actual output image of the tooth segmentation model 500 described with reference to FIG. 6, and FIG. 8 is an exemplary view illustrating an ideal output image of the tooth segmentation model 500 described with reference to FIG. 7.

FIG. 7 illustrates an input image 610 of the tooth segmentation result model 500 trained to identify only the tooth region and the gingiva region and an actual output image 620 corresponding to the input image 610. Here, the input image 610 includes a tooth region 610a, a gingiva region 610b, and a soft tissue region 610c, and similarly, the actual output image 620 includes a tooth region 620a, a gingiva region 620b, and a soft tissue region 620c. In addition, the ideal output image 630 illustrated in FIG. 8 also includes a tooth region 630a, a gingiva region 630b, and a tissue region 630c.

Comparing FIG. 7 and FIG. 8, the actual output image 620 and the ideal output image 630 differ from each other in their soft tissue regions 620c and 630c, and as described above, it can be construed as a result that the soft tissue regions 620c and 630c, which can be a type of a noise region, are represented in attributes similar to those of the gingiva regions 620b and 630b on the two-dimensional image. Due to the soft tissue region 620c of the actual output image 620, a region not intended by the user is identified in the two-dimensional image, and accordingly, a region not intended by the user may also be included in the three-dimensional image. As operations for compensating such a limitation, detailed operations of processing the two-dimensional image with reference to the tooth region in which the accuracy of the identification can be secured due to the distinctiveness of the attribute presented in the two-dimensional image are described with reference to FIGS. 9 and 10.

FIG. 9 is an exemplary view illustrating a two-dimensional image of a target oral cavity, which can be referred to in some embodiments of the present disclosure, and FIG. 10 is an exemplary view illustrating in detail the operation of identifying a first neighboring region, described with reference to FIG. 5, as the operation performed on the two-dimensional image of the target oral cavity, described with reference to FIG. 9.

The input image 610 illustrated in FIG. 9 includes a tooth region 610a, a gingiva region 610b, and a soft tissue region 610c, and an output image 640 in which a tooth region 640a is identified to correspond to the input image 610 is illustrated in FIG. 10. Referring to FIG. 10, it can be understood that first neighboring regions 643a and 643b located within a predetermined distance can be identified with reference to a boundary 641 of the identified tooth region 640a. Here, FIG. 10 illustrates an example in which two first neighboring regions 643a and 643b are identified, and is merely provided for convenience of description, and the number of the first neighboring regions 643a and 643b which can be identified with reference to the boundary 641 of the tooth region 640a may vary. That is, all of the first neighboring regions 643a and 643b located within a predetermined distance with reference to the boundary 641 of the tooth region 640a can be identified, and only some of them may be identified. The selection of all or some of the first neighboring regions by the user 10 is a selection by the user 10 in a trade-off relationship between a consumption of computing resources and the accuracy of image processing to be performed below, and the user 10 may select either one according to an actual implementation case.

Hereinafter, referring to FIG. 11, a more detailed explanation of operation S130 of FIG. 5 is provided below. FIG. 11 is an exemplary flow chart specifically illustrating the operation of determining a region of interest described with reference to FIG. 5.

Referring to FIG. 11, in operation S131, a difference in depth may be calculated by comparing a first coordinate corresponding to a tooth region and a second coordinate corresponding to a first neighboring region. Here, each of the first coordinate and the second coordinate may be a coordinate obtained by using the intraoral scanner 200 linked to the electronic device 100. As described with reference to FIG. 4, when a three-dimensional coordinate is obtained using the intraoral scanner 200, a difference in depth between the respective regions may be calculated using a value (e.g., the first coordinate and the second coordinate) represented on an axis corresponding to a scanning direction of the intraoral scanner 200 among the obtained three-dimensional coordinates.

The coordinates corresponding to the respective regions described above may be obtained in various methods. In some embodiments, the coordinates corresponding to the respective regions may be obtained based on a position of a first camera provided in the intraoral scanner 200, a position of a second camera distinguished from the first camera, and respective images captured by the cameras. Specifically, by comparing and analyzing respective images captured by a left camera (e.g., the first camera) corresponding to a left eye field and a right camera (e.g., the second camera) corresponding to a right eye field according to an optical triangulation method with the positions of the cameras, the coordinates corresponding to the respective regions may be obtained. However, regarding a detailed operation using the optical triangulation method, a detailed description is omitted so as not to blur the point of the present disclosure. In some other embodiments, the coordinates corresponding to the respective regions may be obtained through monocular depth estimation for the two-dimensional image captured from the intraoral scanner 200. Here, the monocular depth estimation is a three-dimensional depth estimation method using a two-dimensional image captured by a single camera. In a specific example, a three-dimensional image is restored from a two-dimensional image by using a DenseDepth model, the depth of a region on the two-dimensional image can be estimated accordingly, and in addition to the examples mentioned above, all known methods of estimating a depth of a two-dimensional image through a two-dimensional image captured by a single camera may be applied to the present disclosure.

Next, when the difference in depth is equal to or greater than a threshold (S132), in operation S133, the first neighboring region may be excluded from the region of interest, and when the difference in depth is less than the threshold (S132), in operation 134, the first neighboring region may be included in the region of interest. Here, the threshold is a type of a configuration value which can be a reference for exclusion from and inclusion in the region of interest, and the user 10 may change the threshold according to an actual implementation case. When the gingiva region is included in the region of interest and the soft tissue region is excluded from the region of interest, a proper threshold which can distinguish two regions may be obtained through experiment.

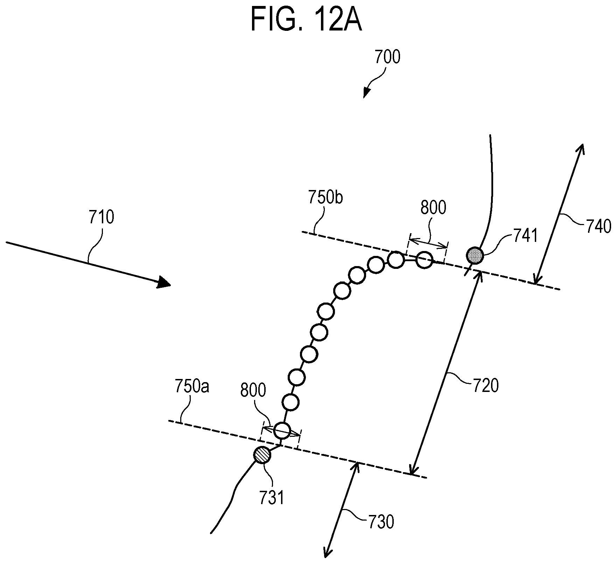

Hereinafter, referring to FIGS. 12A to 12D, a detailed description of the operation of determining a region of interest described with reference to FIG. 11 is made. FIGS. 12A to 12D are exemplary views illustrating in detail the operation of determining a region of interest, described with reference to FIG. 11.

FIG. 12A is an example of a virtual sectional image 700 of the subject 20, and illustrates a scanning direction 710, a tooth region 720, a gingiva region 730, and a soft tissue region 740 of the intraoral scanner 200. Here, it can be understood that the tooth region 720 is distinguished from a first boundary 750a from the gingiva region 730 and a second boundary 750b from the soft tissue region 740. As described above with reference to FIG. 10, first neighboring regions 731 and 741 may be identified from the boundaries 750a and 750b of the tooth region 720, respectively. In addition, as described above with reference to FIG. 11, a difference in depth between a first coordinate corresponding to the tooth region 720 and a second coordinate corresponding to the first neighboring regions 731 and 741 may be calculated. Here, the first coordinate corresponding to the tooth region 720 may be a coordinate of a minute region included in a tooth region within the closest distance from the first neighboring regions 731 and 741, and referring to FIG. 12A, it can be understood that the depths between the minute regions in the closest distance from the respective first neighboring regions 731 and 741 and the first neighboring regions 731 and 741 are compared. According to the example illustrated in FIG. 12A, a difference in depth between the first neighboring region 731 located in the gingiva region 730 and the tooth region 720 is less than a threshold 800, and a difference in depth between the first neighboring region 741 located in the soft tissue region 74 and the tooth region 720 is equal to or greater than the threshold 800. Accordingly, the first neighboring region 741 located in the soft tissue region 740 is excluded from the region of interest, and the first neighboring region 731 located in the gingiva region 730 is included in the region of interest. As described above with reference to FIG. 11, by configuring a proper threshold 800, the operation of determining the region of interest may be performed as in the example illustrated in FIG. 12A.

The operation of determining the region of interest may be performed as the region of interest gradually expands according to a predetermined rule. Hereinafter, a more detailed description of the expansion of the region of interest will be provided.

With regard to operation S133 illustrated in FIG. 11, in some embodiments, the operation of excluding the first neighboring region from the region of interest when the difference in depth from the tooth region is equal to or greater than a threshold may include an operation of excluding a second neighboring region from the region of interest even though the difference in depth between the first neighboring region and the second neighboring region located within a predetermined distance from the boundary of the first neighboring region is less than the threshold. Here, with regard to the operation of identifying the second neighboring region, the description of operation S 120 in FIG. 5 may be referred to, and for the operation of calculating the difference in depth, the description of FIG. 11 may be referred to.

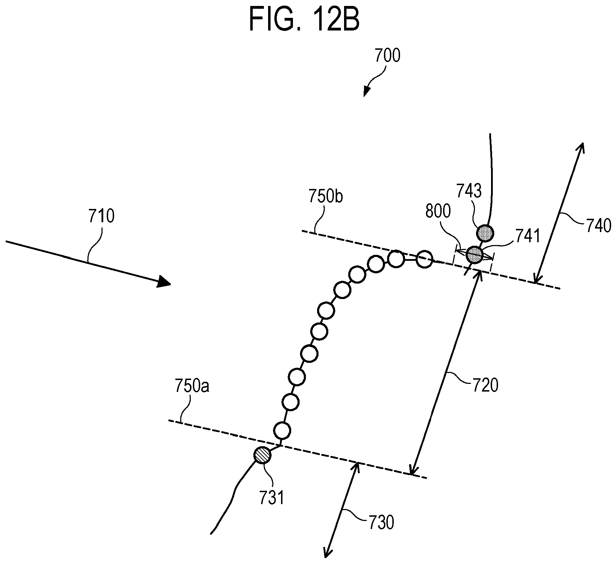

Referring to FIG. 12B for describing the present embodiment in more detail, it can be understood that even though a difference in depth between a first neighboring region 741 located in a soft tissue region 740 and a second neighboring region 743 located in the soft tissue region is less than a threshold 800, a second neighboring region located in the soft tissue region 740 is excluded from the region of interest. That is, the rule described here is a rule disallowing incorporation of a region expanded from the boundary of the region excluded once (e.g., the first neighboring region 741 located in the soft tissue region 740) into the region of interest, and through such a rule, computing resources unnecessarily consumed for operation for the region which cannot be included in the region of interest can be saved.

With regard to operation S 134 illustrated in FIG. 11, in some embodiments, including the first neighboring region in the region of interest when the difference in depth from the tooth region is less than the threshold may include repeatedly expanding the region of interest until the difference in depth between the region of interest and a third neighboring region located within a predetermined distance from the boundary of the region of interest becomes equal to or greater than the threshold. Here, for the operation of identifying the third neighboring region, the description of operation S 120 in FIG. 5 may be referred to, and for the operation of calculating the difference in depth, the description of FIG. 11 may be referred to.

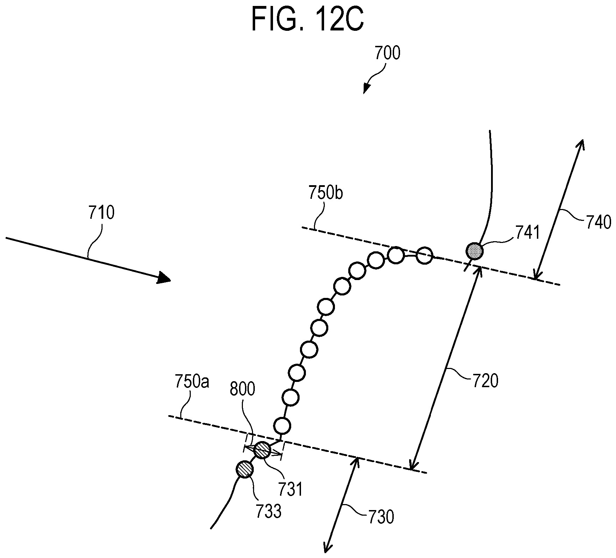

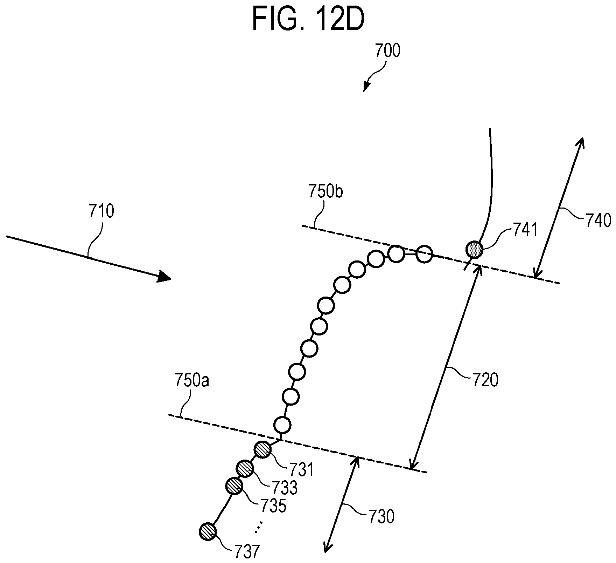

Referring to FIG. 12C for describing the present embodiment in more detail, a first neighboring region 731 located in a gingiva region 730 is a region included in the region of interest, as described with reference to FIG. 12A. Accordingly, the first neighboring region 731 located in the gingiva region 730 may be understood as a minute region corresponding to the region of interest, and whether to include a third neighboring region 733 located in the gingiva region 730 in the region of interest may be determined through comparison of a difference in depth between the first neighboring region 731 located in the gingiva region 730 and the third neighboring region 733 located in the gingiva region 730. According to the example illustrated in FIG. 12C, since the difference in depth between the region of interest (e.g., the first neighboring region 731 located in the gingiva region 730) and the third neighboring region 733 located in the gingiva region 730 is less than a threshold 800, it may be understood that the third neighboring region 733 located in the gingiva region 730 is included in the region of interest. Similarly, according to the rule, the region of interest can be repeatedly expanded, and referring to FIG. 12D, it can be understood that minute regions 731, 733, 735, and 737 of the gingiva region are repeatedly included in the region of interest. That is, the rule described herein is a rule of repeatedly expanding the region of interest by repeatedly applying the same rule corresponding to the comparison of depth between the expanded region of interest and the neighboring region thereof, and through such a rule, the region of interest can be determined to satisfy the intention of the user 10.

To describe the above-described operation of expanding the region of interest with a specific example, gingiva regions consecutively connected from a tooth region may be incorporated into a region of interest, and a noise region (e.g., a soft tissue region, etc.) having a distance from the tooth region may be excluded from the region of interest. In the above-described operation of expanding the region of interest, the distal end of the region of interest is determined by a single rule of calculating a difference in depth between the region to be expanded (e.g., the third neighboring region) and the region of interest, but hereinafter, other operations of determining the distal end of the region of interest are described.

In some embodiments, in the operation of repeatedly expanding the region of interest, even though the difference in depth between the region of interest and the third neighboring region is less than the threshold, the expansion of the region of interest can be suspended when an expansion count is equal to or greater than a reference count. Here, the reference count is a type of a configuration value which can be a reference for exclusion from and inclusion in the region of interest, and the user 10 may change the reference count according to an actual implementation case. In a case of including only a predetermined part of the gingiva region in the region of interest, a proper reference count may be obtained through experiment. According to the present embodiment, the region of interest may be determined to satisfy the intention of the user, and for example, even in a case of the gingiva region, only a partial region of the gingiva region necessary for dental treatment may be included in the region of interest. In addition, computing resources consumed by the operation for a region unnecessary for dental treatment can be saved.

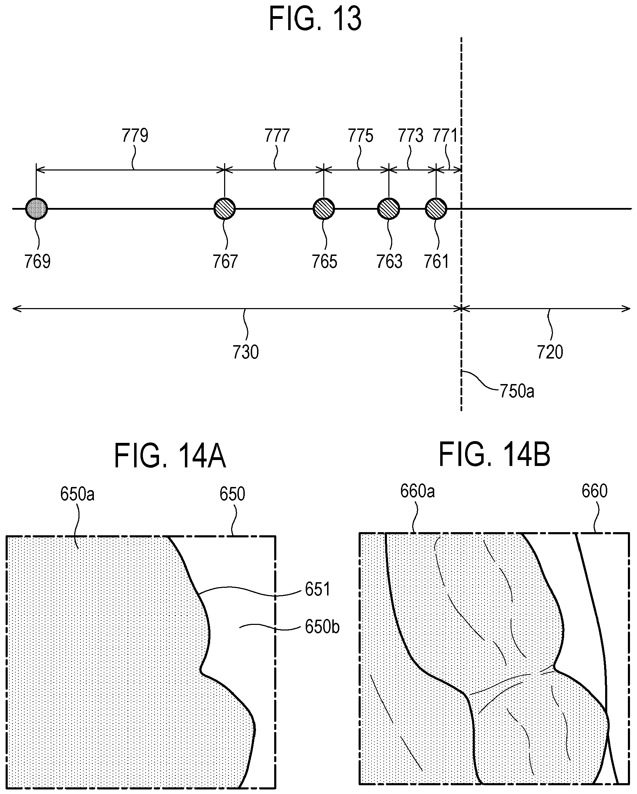

In some other embodiments, when the region of interest is repeatedly expanded, the distance between the boundary of the region of interest and the region to be expanded (e.g., the third neighboring region) may increase as the expansion count increases. Here, the margin of increase in the distance is a type of a configuration value which can be a reference for exclusion from and inclusion in the region of interest, and the user 10 may change the margin of increase in the distance according to an actual implementation case. In a case of including only a predetermined part of the gingiva region in the region of interest, a proper margin of interest in the distance may be obtained through experiment. A detailed description related thereto is provided below with reference to FIG. 13.

FIG. 13 is an exemplary view illustrating in detail an operation of expanding a region of interest and an operation of determining a neighboring region upon the expansion, which can be referred to in some embodiments of the present disclosure. FIG. 13 is an example of a virtual sectional image obtained by simplifying the oral cavity of the subject 20, and illustrates an example in which a tooth region 720 and a gingiva region 730 are divided by a first boundary 750a. Here, it can be identified that the distance 771, 773, 775, 777, or 779 between the boundary of the region of interest and the region to be extended 761, 763, 765, 767, or 769 increases as the extension count increases, and in this case, as described with reference to FIGS. 12A to 12D, whether to include the region to be extended 761, 763, 765, 767, or 769 in the region of interest may be determined by calculating a difference in depth in a scanning direction 710. As in the example illustrated in FIG. 13, even though all the regions to be expanded 761, 763, 765, 767, and 769 are included in the gingiva region, it can be understood that first minute regions 761, 763, 765, and 767 are included in the region of interest and a second minute region 769 is excluded from the region of interest. According to the present embodiment, the region of interest may be determined to satisfy the intention of the user, and for example, even in a case of the gingiva region, only a partial region of the gingiva region necessary for dental treatment may be included in the region of interest. In addition, computing resources consumed by the operation for a region unnecessary for dental treatment can be saved. Specifically, the distance 771, 773, 775, 777, or 779 between the boundary of the region of interest and the region to be expanded 761, 763, 765, 767, or 769 increases as the expansion count increases, and thus the distal end of the region of interest may be determined more promptly by calculating a smaller number of expansion counts than that in a case where the distance between the boundary of the regio and the region to be expanded remains the same as the region of interest is expanded.

The operations of determining the distal end of the region of interest are described above. According to the various rules described above, computing resources can be saved while determining the region of interest satisfying the intention of the user. Hereinafter, a specific example of a region of interest determined according to the above-described operations is described with reference to FIG. 14A.

FIG. 14A is an exemplary view illustrating a region of interest determined for a two-dimensional image of a target oral cavity as described with reference to FIG. 9. FIG. 14A illustrates an example of an output image 650 corresponding to the input image 610 illustrated in FIG. 9, and the output image 650 illustrated in FIG. 14A includes a region of interest 650a. Comparing FIG. 9 and FIG. 14A, it can be understood that on the output image 650, the region of interest 650a (corresponding to the tooth region 610a and the gingiva region 610b of the input image 610) and a noise region 650b (corresponding to the soft tissue region 610c of the input image 610) are divided by a boundary 651 of the region of interest 650a.

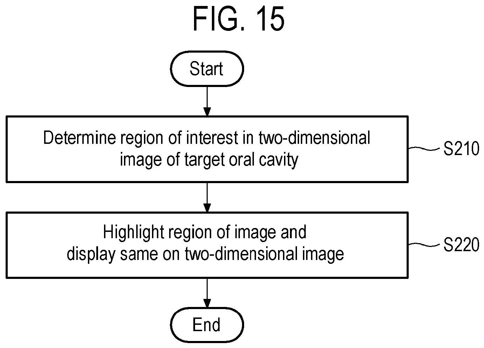

According to the image processing method according to some embodiments of the present disclosure described with reference to FIG. 5, FIG. 11, and related exemplary views, a region of interest of a two-dimensional image, which is necessary for dental treatment, i.e., which is an object of interest of the user 10, may be dynamically determined, and a three-dimensional image may be generated based on the determined two-dimensional image. In addition, according to the above-described image processing method, a high-quality three-dimensional image (i.e., an image including less noise) may be generated while minimizing the involvement of the user 10. Hereinafter, with reference to FIGS. 15 to 19, other exemplary operations illustrating the image processing method according to some embodiments of the present disclosure will be described.

First, FIG. 15 is another exemplary flow chart illustrating an image processing method according to some embodiments of the present disclosure. Referring to FIG. 15, in operation S210, a region of interest may be determined in a two-dimensional image of a target oral cavity. This operation can be understood with reference to the description of operations S110, S120, and S130 of FIG. 5.

Next, in operation S220, the region of interest on the two-dimensional image may be highlighted and displayed. Here, for highlighting of the region of interest, various known graphic visualization technologies may be referred to. For example, as in the output image 660 illustrated in FIG. 14B, a mask 660a may be overlaid over the region of interest, but the scope of the present disclosure is not limited thereto, and any technologies which enable the user 10 to visually recognize the determined region of interest and any methods of highlighting the region of interest may be included in the scope of the present disclosure.

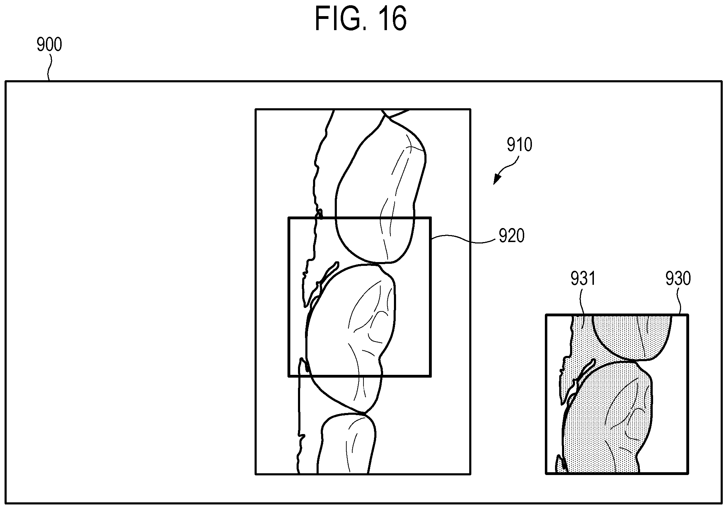

In some embodiments, an output image obtained by highlighting the region of interest and a three-dimensional image corresponding to the output image may be displayed together. For example, as in a screen 900 of the electronic device 100 illustrated in FIG. 16, an output image 930 obtained by highlighting a region of interest 931 and a three-dimensional image 910 corresponding to the output image may be displayed in one screen 900. Here, a reference box 920 may be further displayed on the screen 900 to enable the user 10 to easily recognize a region on the three-dimensional image 910 corresponding to the output image 930 output on the screen 900.

According to the image processing method according to some embodiments of the present disclosure described with reference to FIG. 15 and related exemplary views, the user 10 may visually identify a region of interest utilized for conversion into a three-dimensional image. As the user 10 visually identifies such a region of interest, the user 10 may identify an error which may occur in determining the region of interest, and may correct the error through the user interface described above with reference to FIG. 2. Specifically, as the user 10 identifies an output image obtained by highlighting a region of interest and a three-dimensional image corresponding to the output image together in one screen, the user 10 may identify in more detail an error which may occur in determining the region of interest. In addition, after completion of scanning or during the scanning process, the user 10 performs dental treatment while identifying a region of interest of a two-dimensional image displayed on the screen of the electronic device 100, and thus the user convenience can be maximized.

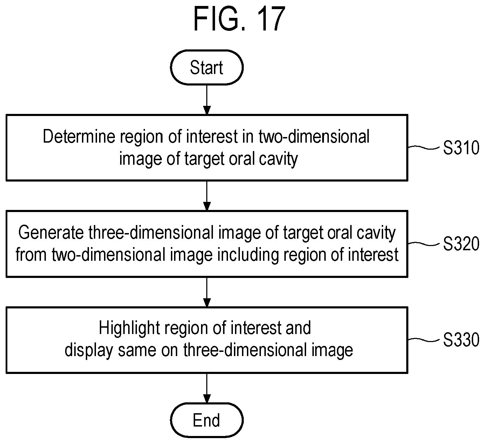

Next, FIG. 17 is another exemplary flow chart illustrating an image processing method according to some embodiments of the present disclosure. Referring to FIG. 17, in operation S310, a region of interest may be determined in a two-dimensional image of a target oral cavity. This operation can be understood with reference to the description of operations S110, S120, and S130 of FIG. 5. Next, in operation S320, a three-dimensional image of the target oral cavity may be generated from the two-dimensional image including the region of interest. This operation can be understood with reference to the description of operation S140 of FIG. 5.

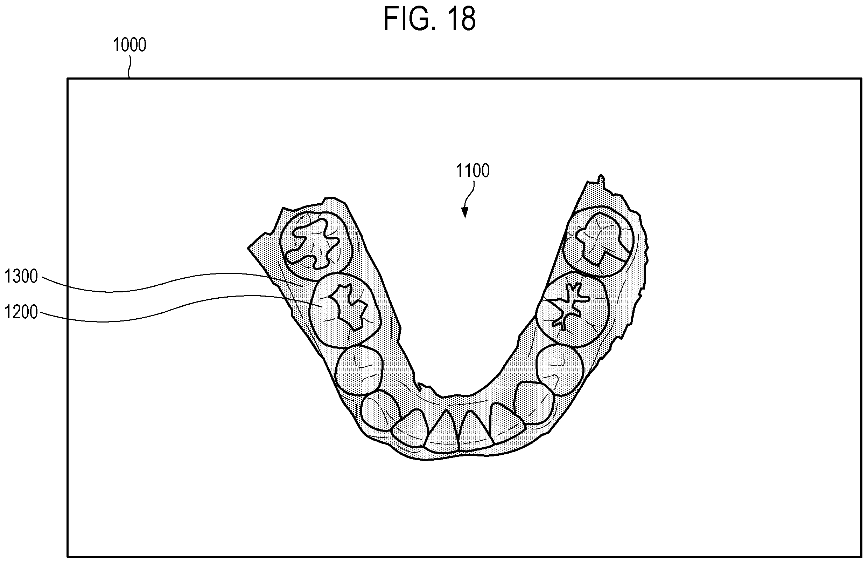

Next, in operation S330, the region of interest may be highlighted and displayed on the three-dimensional image. Here, for highlighting the region of interest, various known graphic visualization technologies may be referred to. For example, as in a screen 1000 including a three-dimensional image 110 illustrated in FIG. 18, a mask may be overlaid over the region of interest including a tooth region 1200 and a gingiva region 1300. The scope of the present disclosure is not limited to this example, and any technologies which enable the user 10 to visually recognize the determined region of interest and any methods of highlighting the region of interest may be included in the scope of the present disclosure.

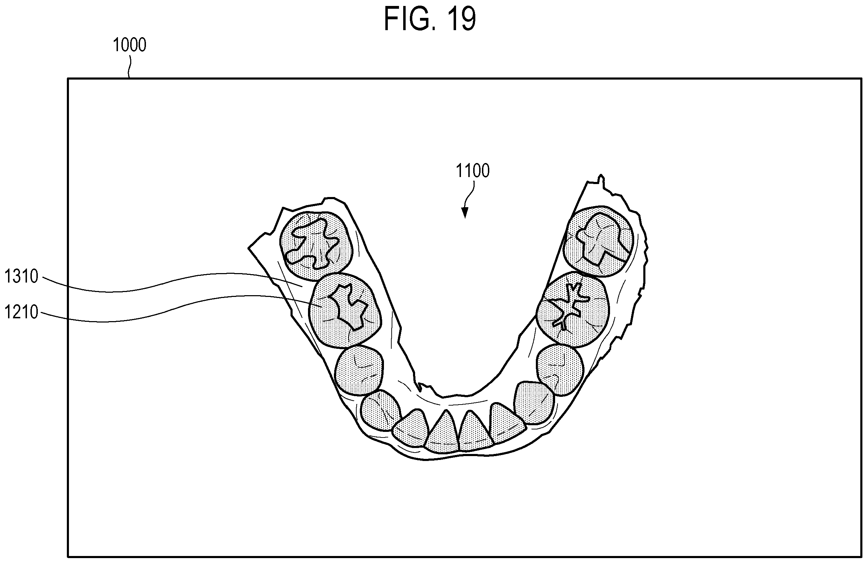

In some embodiments, a tooth region which is more essential for dental treatment may be highlighted and displayed on the three-dimensional image. For example, as in the screen 1000 including a three-dimensional image 1100 illustrated in FIG. 19, a mask may be overlaid over a tooth region 1210 and may not be overlaid over a gingiva region 1310, but the scope of the present disclosure is not limited thereto, and any technologies which enable the user 10 to visually recognize the tooth region and any methods of highlighting the tooth region may be included in the scope of the present disclosure.

According to the image processing method according to some embodiments of the present disclosure described with reference to FIG. 17 and related exemplary views, the user 10 may visually identify a region of interest on a three-dimensional image. The user 10 may identify a region which is essential for dental treatment by visually identifying such a region of interest. That is, after completion of scanning or during the scanning process, the user 10 performs dental treatment while identifying a region of interest of a two-dimensional image displayed on the screen of the electronic device 100, and thus the user convenience can be maximized.

With reference to FIGS. 1 to 19, various embodiments of the present disclosure and effects according to the embodiments are mentioned. The effects according to the technical spirit of the present disclosure are not limited to the above-mentioned effects, and other unmentioned effects can be clearly understood by those skilled in the art from the description above.

The technical spirit of the present disclosure described with reference to FIGS. 1 to 19 may be implemented as computer-readable code on a recording medium readable by a computing device (e.g., electronic device). Here, a computer program implemented as code may include, when being loaded to a memory of the computing device, at least one instruction which causes a processor of the computing device to perform operations according to various embodiments of the present disclosure. Such a computer-readable recording medium may be, for example, a removable recording medium (e.g., CD, DVD, Blue-ray disk, USB drive, removable hard disk, etc.) or a fixed recording medium (e.g., ROM, RAM, built-in hard disk, etc.). In addition, the recording medium may be a non-transitory recording medium. Here, the non-transitory recording medium refers to a tangible medium regardless of whether data is stored semi-permanently or temporarily, and does not include a temporarily transmitted signal. Moreover, the computer program recorded in the recording medium may be transmitted to another computing device via a network such as Internet and installed in another computing device, and may be thus used in other computing devices.

Although the technical spirit of the present disclosure has been described by the examples described in some embodiments and illustrated in the accompanying drawings, it should be noted that various substitutions, modifications, and changes can be made without departing from the scope of the present disclosure which can be understood by those skilled in the art to which the present disclosure pertains. In addition, it should be noted that that such substitutions, modifications and changes are intended to fall within the scope of the appended claims.

## Claims

1. An image processing method performed by an electronic device, the method comprising:
identifying a tooth region in a two-dimensional image of a target oral cavity;
identifying, in the two-dimensional image, a first neighboring region located within a predetermined distance from a boundary of the tooth region;
determining, based on a difference in depth between the tooth region and the first neighboring region, whether to include the first neighboring region in a region of interest; and
generating a three-dimensional image of the target oral cavity from the two-dimensional image which includes the region of interest.

2. The method of Claim 1, wherein the identifying the tooth region comprises identifying the tooth region in the two-dimensional image by using a tooth segmentation model constructed according to a machine learning algorithm, and
wherein the tooth segmentation model corresponds to a model trained by modeling a correlation between a training image set of a tooth and a segmentation result image set corresponding to the training image set.

3. The method of Claim 1, wherein the determining whether to include the first neighboring region in the region of interest comprises comparing a first coordinate corresponding to the tooth region with a second coordinate corresponding to the first neighboring region to calculate the difference in depth, and
wherein each of the first coordinate and the second coordinate corresponds to a coordinate obtained using an intraoral scanner linked to the electronic device.

4. The method of Claim 3, wherein each of the first coordinate and the second coordinate is a coordinate calculated based on a position of a first camera, a position of a second camera distinguished from the first camera, an image captured by the first camera, and an image captured by the second camera, and
wherein the first camera and the second camera are cameras provided in the intraoral scanner.

5. The method of Claim 3, wherein each of the first coordinate and the second coordinate is a coordinate obtained through monocular depth estimation of the two-dimensional image captured from the intraoral scanner.

6. The method of Claim 1, wherein the determining whether to include the first neighboring region in the region of interest comprises excluding the first neighboring region from the region of interest when the difference in depth is equal to or greater than a threshold.

7. The method of Claim 6, wherein the excluding the first neighboring region from the region of interest comprises even when a difference in depth between the first neighboring region and a second neighboring region located within the predetermined distance from the boundary of the first neighboring region is less than the threshold, excluding the second neighboring region from the region of interest.

8. The method of Claim 1, wherein the determining whether to include the first neighboring region in the region of interest comprises when the difference in depth is less than a threshold, including the first neighboring region in the region of interest.

9. The method of Claim 8, wherein the including the first neighboring region in the region of interest comprises repeatedly expanding the region of interest until the difference in depth between the region of interest and a third neighboring region located within the predetermined distance from the boundary of the region of interest becomes equal to or greater than the threshold.

10. The method of Claim 9, wherein a distance between the boundary of the region of interest and the third neighboring region increases as an expansion count increases.

11. The method of Claim 9, wherein the repeatedly expanding of the region of interest comprises when an expansion count is equal to or greater than a reference count, suspending an expansion of the region of interest even when the difference in depth between the region of interest and the third neighboring region is less than the threshold.

12. The method of Claim 1, further comprising displaying the region of interest by highlighting the region of interest on the two-dimensional image.

13. The method of Claim 1, further comprising displaying the region of interest by highlighting the region of interest on the three-dimensional image.

14. An electronic device comprising:
a processor;
a network interface communicatively connected to an intraoral scanner;
a display;
a memory; and
a computer program loaded onto the memory and executed by the processor,
wherein the computer program comprises instructions for:
identifying a tooth region in a two-dimensional image of a target oral cavity;
identifying, in the two-dimensional image, a first neighboring region located within a predetermined distance from a boundary of the tooth region;
determining, based on a difference in depth between the tooth region and the first neighboring region, whether to include the first neighboring region in a region of interest; and
generating a three-dimensional image of the target oral cavity from the two-dimensional image which includes the region of interest.

15. The electronic device of Claim 14, wherein the instruction for determining whether to include the first neighboring region in the region of interest comprises an instruction for when the difference in depth is equal to or greater than a threshold, excluding the first neighboring region from the region of interest, and when the difference in depth is less than the threshold, including the first neighboring region in the region of interest.

16. The electronic device of Claim 14, wherein the computer program further comprises an instruction for displaying the region of interest by highlighting the region of interest on the two-dimensional image.

17. The electronic device of Claim 14, wherein the computer program further comprises an instruction for displaying the region of interest by highlighting the region of interest on the three-dimensional image.

18. A non-transitory computer-readable recording medium in which a computer program to be executed by a processor is recorded,
wherein the computer program comprises instructions for:
identifying a tooth region in a two-dimensional image of a target oral cavity;
identifying, in the two-dimensional image, a first neighboring region located within a predetermined distance from a boundary of the tooth region;
determining, based on a difference in depth between the tooth region and the first neighboring region, whether to include the first neighboring region in a region of interest; and
generating a three-dimensional image of the target oral cavity from the two-dimensional image which includes the region of interest.

19. The computer-readable recording medium of Claim 18, wherein the computer program further comprises an instruction for displaying the region of interest by highlighting the region of interest on the two-dimensional image.

20. The computer-readable recording medium of Claim 18, wherein the computer program further comprises an instruction for displaying the region of interest by highlighting the region of interest on the three-dimensional image.
